# EUROPEAN PATENT APPLICATION

(11) **EP 1 705 513 A1**
(43) Date of publication of application: **27.09.2006**
(21) Application number: 03780506.6
(22) Date of filing: 19.12.2003
(51) Int. Cl.: G02B 27/22, A61B 1/00

(54) **SYSTEM FOR THE STEREOSCOPIC VIEWING OF REAL-TIME OR STATIC IMAGES**

(71) Applicant: De Font-Réaulx-Rojas, Enrique, 09300 Mexico D.F. (MX)
(72) Inventor: De Font-Réaulx-Rojas, Enrique, 09300 Mexico D.F. (MX)
(74) Representative: Temino Ceniceros, Ignacio
(86) International application number: PCT/IB2003/006284
(87) International publication number: WO 2005/066690

(57) **Abstract**

The invention relates to a system for the three-dimensional stereoscopic viewing of real-time or static images, which displays a real image that is obtained from image-capture means at the moment at which said images are generated with a simple and very effective three-dimensional viewing effect, thereby enabling the observer to perform fine, precise movements with an adequate perception of volume, distance and depth.

## Description

### DOMAIN OF THE INVENTION

The invention herein is related to the technique of viewing three-dimensional images, and more particularly, it is related to a system for the stereoscopic viewing of real time or static images.

### HISTORY OF THE INVENTION

Both in the medical field and in certain industrial applications, it is useful to stereoscopically view images obtained by various means. This is the case in endoscopy, which is used for inspecting and manipulating structures of interest in locations that are not accessible by any other means or whose access involves the possibility of causing injury to the patients in case of their medical application.

In the medical field, the main interest is that of reducing trauma in patients, facilitating postoperative care, and reducing the hospital-stay time with better surgical results, known as Minimally Invasive Surgery (MIS), and it has developed in practically all medical-surgical specialties.

Currently, the different endoscopy systems are widely used in neurosurgery in the so-called "endoscopy-assisted cerebral surgeries" and in the minimally invasive techniques, because they provide better illumination of the surgical area since it is possible to illuminate and view locations of the surgery area where the straight light of the operating theater lamps or of surgical microscope does not have access. With the current development in the design of endoscopy instruments in all their varieties, it is possible to perform complete surgical techniques by having instruments available for cutting, coagulation, tumor vaporization, lavage, perforation, dissection by means of radio frequency, laser and instruments for guiding and orientation.

In the MIS procedures instruments are introduced into the body through small incisions or by percutaneous intubation for performing surgical procedures while avoiding the need of making the large incisions necessary in open surgical procedures. As described above, viewing is facilitated by using special instruments called endoscopes, laparoscopes, neuroendoscopes, arthroscopes and other varieties related to the same function, which are conventionally rigid or flexible tubular instruments that contain a system of lenses or optical fiber, and in their proximal portion, a part that allows a direct monocular view or the installation of a video camera. The distal portion of the instrument is introduced in the desired region, and the surgeon can observe the inside of body cavities, either by direct viewing in the monocular of the endoscope, or by being shown on a video monitor. Usually, these instruments include a light source for illumination of the body cavity.

As the complexity of the procedures that can be performed by minimally invasive surgery techniques advances, it becomes ever more important to have adequate perception and a precise proportion of the distance between the objects subject to direct viewing, as this occurs in the so-called "open sky" surgeries, in which the surgeon performs the procedure subject to direct viewing while maintaining three-dimensional stereoscopic viewing. However, in the MIS procedures, in which conventional endoscopy systems are used, this visual distance and depth information is lost because some of these systems are for monocular viewing or for two-dimensional viewing showing the images on video monitors, liquid crystal or plasma displays, in spite of the high image quality that the last two can show.

Because of this disadvantage, different endoscopy systems have recently been developed with the objective of improving the image quality and of providing this important distance and depth information. The main conventional endoscopy systems are the following: The straight "Lenscope" provides better optical resolution and illumination than the rest of the endoscopes; the Hopkins model permits wide viewing angles with good image quality, its size can be up to 1 mm in diameter, which permits recognizing even anatomic structures; the "Fiberscope" has the advantage that it permits directing its tip, but it has the disadvantage that, when its size is reduced, the image is deformed; it is particularly useful for the simultaneous use with the surgical microscope for inspecting places where the straight light of the microscope does not manage to permit their proper viewing.

Among the "videoscopes and stereovideoscopes", commercially there are stereoendoscopes of up to 14 mm in diameter that provide true stereoscopic field depth of the surgical environment by means of two photosensitive chips in the tip (Medical Dynamics, Inc. Englewood, CO), which represents an enhancement as far as safety in performing dissections and movements is concerned in comparison to the monoscopic field that the lenscope and the fiberscope offer, because this model is capable of offering a feeling of a three-dimensional image; however, in the special case of neurosurgery, these stereoendoscopes currently have a diameter that is unacceptable for being introduced into the cranial cavity.

The current main limitations of conventional endoscopy are: 1) Its high cost; 2) when observing the image on a remote monitor in the surgical area, it interferes with "eye-hand" coordination; 3) it is two-dimensional, and the surgeon may lose the perception of the distances between viewed objects, which sometimes makes the procedures difficult, and this limitation may cause complications in endoscopic procedures; 4) currently it is limited as a sole surgical technique for procedures that require great precision in the movements of the surgical instruments (Example: clipping of intracranial aneurisms).

Three-dimensional neuroendoscopes currently have as disadvantages: 1) some models have too wide a diameter to be introduced into tight body cavities, such as the intracranial cavities; 2) other recent models do not offer a real image but instead a "virtual reality" type image that is very different from the human anatomy, or else do not transmit in real time; 3) high cost; 4) use of very sophisticated technology; 5) special training is required; 6) low quality of the three-dimensional effect.

The Virtual Reality (VR) systems with medical-surgical application are still in an experimental stage as prototypes or projects. They can be classified as diagnostic, diagnostic-therapeutic, for rehabilitation, tutorial or research. Some may be designed and used at a distance (remote presence, remote robotic surgery) for direct or simulated use. Other VR systems may be connected in networks, to be used with VR gloves or VR visors, including the so-called "3D endoscopy", which is performed using a video system that includes 3-D animation and photographs which involve the disadvantage of not being compatible with the rigid halo systems such as those of stereotactic neurosurgery, the clinical utility of which remains to be defined, whose post-processing procedure induces a variety of significant errors related to the system operator and the images of which that the observer receives are the product of a computer-simulated animation system, hence being artificial and very different from the real appearance of the patient's anatomy, in spite of the variable degree of precision that they may have.

Some MIS procedures require the surgeon's constant work for several hours, a period of time, during which the latter is forced to keep the view fixed or to adopt uncomfortable positions during extended periods of time, which may have an effect on the results of the procedure. There are some devices that make the performance of these procedures more comfortable for the surgeon, such as the one that shows the image on a video monitor instead of by direct monocular viewing on the endoscope. Recently a system was developed for projecting cephalic placement video images, in which the surgeons can observe both the endoscopy and the surgical microscopy images and those of the image studies while requesting them orally, conveniently and with the great advantage of maintaining the "eye-hand" relation and achieve more precise control of their movements. However, these systems show the images two-dimensionally and lose the proportion of the distance and depth of objects in the surgical area.

It will be observed from the description above that so far it has not been successful to develop a system that, in addition to viewing images obtained by means of the various instruments, permits obtaining a stereoscopic view that permits surgeons to properly coordinate their hands and what the eyes observe, and thus to have a concept of the proper depth that permits performing high precision surgeries in real time, in spite of the existence of various systems that permit obtaining stereoscopic images.

In this respect, in the state of the art, there is British Patent Series No. GB 784,919 which refers to the enhancements achieved in a system for stereoscopic viewing using a pair of images: a "mirror-like" inverted image, while the other image is not modified. To observe the images simultaneously, the observer looks through some orifices placed in front of a mirror at 45°. Placing the stereoscopic images on a plane that is not horizontal produces a system that is not very practical because by being placed on a cephalic fixation device, it does not permit the surgeon to have a peripheric view.

The same disadvantage is evident in British Patents Series Nos. GB 2,052,088 and GB 2,131,969 645 296, as well as in the British Patent registered under number GB 2,221,054-A, which uses two different angles, all of them having the common denominator of having a mirror or a reflecting surface located so as to coincide with the line bisecting this angle.

In turn, British Patent Series No. GB 2 312 966A uses an angle de 180° between the images. However, the device described here requires the normal and "mirror" images to be generated in advance, which prevents their use in images generated in real time.

In order to manage to overcome these disadvantages, in International Patent Application No. PCT/MX02/00047, whose inventor is the same as the one of the invention herein, a stereoscopic viewing system is described, which is formed by capturing images that capture an original image, either with or without movement; means for duplicating and inverting images in combination with means of converting images to digital and/or analog signals, which, as a result of their combined effect, simultaneously generate a duplicated image together with a mirror-like inverted image and/or a duplicated image signal together with an inverted image which correspond to these duplicated and inverted images respectively; and three-dimensional viewing means that receive the duplicated and inverted image signals to achieve three-dimensional viewing of the original image by means of the combination of the duplicated and inverted images.

However, the system involves the disadvantage that it is a fairly complex system, since one of its characteristics is that, in the image duplication and inversion means, various components are used such as conductors, reflecting and/or translucent surfaces, lenses and adaptors for obtaining both the inverted image and the duplicated image. In a similar manner, in the means of three-dimensional viewing, a reflecting surface is used placed between a first and a second image projection element.

On the other hand, one of the needs that arises when a surgical operation is performed is being able to three-dimensionally observe the surgical area as well as the images of various studies carried out previously. In this respect, none of the viewing systems mentioned and in accordance with the state of the art permit including the information from the transoperative guiding systems, such as the navigators, ultrasound and fluoroscopy, among others, used during the surgeries.

As a consequence of the above, it was attempted to eliminate the disadvantages the currently utilized viewing systems involve by developing a real time or static image viewing system which, in addition to permitting the obtention of high quality three-dimensional images, permits an adequate perception of the volume, distance and depth among the objects observed, using the current conventional endoscopes of the medical-surgical practice. Likewise, it permits the surgeon to carry out fine and precise movements, even in very limited space by modifying only the form in which they are shown on conventional video devices or by means of liquid crystal or plasma screens and maintains the "eye-hand" coordination because the images of the surgical area are shown in their different varieties or those of the studies of the image of the patients by means of a cephalic fixation device that does not include reflecting surfaces, showing in this way the images in a direct way in front of the surgeon's eyes.

### OBJECTIVES OF THE INVENTION

Taking into consideration the defects of the previous technique, one objective of the invention herein is providing a stereoscopic real time or static image viewing system that is very simple and practical, and yet highly efficient, because, in addition to permitting the obtention of high quality three-dintensional images, it permits proper perception of volume, distance and depth among the objects observed.

Another objective of the invention herein is providing a stereoscopic real time or static image viewing system, which permits using the conventional endoscopes in the current medical-surgical practice without making any modification to them, permitting the surgeon to perform fine and precise movements, even in very limited space, by modifying only the form in which they are shown on conventional video devices or by means of liquid crystal or plasma screens.

It is an objective of the invention herein to provide a stereoscopic real time or static image viewing system, which permits the surgeon to maintain "eye-hand" coordination by showing the images of the surgical area in their different varieties or else of the studies of the patient image by means of a cephalic and/or facial placement device that shows the images in front of the surgeon's eyes.

It is yet another objective of the invention herein to provide a stereoscopic real time or static image viewing system that is applicable to the stereotactic surgery systems, with and without frame, and to any processing system of printed images or video images that may be either static or in movement, allowing them to be shown on any type of screen or printed surface, either curved or flat, including the information from transoperative guiding systems, such as navigators, ultrasound, and fluoroscopy, among others, used during the surgeries.

It continues to be another objective of the invention herein to provide a stereoscopic real time or static image viewing system that permits achieving a three-dimensional viewing effect, duplicating the original image to show at least one of these images at a visual angle of incidence different from that of the original image.

It is yet another objective of the invention herein to provide a stereoscopic real time or static image viewing system, in which the images are shown directly in front of a user's eyes by means of cephalic or facial placement means, in which it is not necessary to use reflecting surfaces.

An additional objective of the invention herein is providing a stereoscopic real time or static image viewing system that permits obtaining combinations of images in boxes that can be shown to a user three-dimensionally.

### BRIEF DESCRIPTION OF THE FIGURES

The novel aspects that are considered characteristics of the invention herein will be established in detail in the attached claims. However, the operation, together with its other objectives and advantages, will be better understood from the following detailed description of a specific embodiment, when read with reference to the attached drawings, in which:
Figure 1 shows a block diagram of a stereoscopic real time or static image viewing system where the operational sequence for a specific embodiment of the invention herein is shown.
Figure 2A shows a first configuration of the first image modifying unit used in the invention herein.
Figure 2B shows a second configuration of the first image modifying unit used in the invention herein.
Figure 3 shows a block diagram of the three-dimensional viewing means of the stereoscopic real time or static image viewing system of the invention herein.
Figure 4 is a perspective view of the three-dimensional viewing means used in the invention herein, which are mounted on a cephalic and/or facial placement means.
Figure 5 is a schematic diagram, which shows the visual angles of incidence of an observer positioned in front of the three-dimensional viewing means shown in Figure 4.
Figure 6 shows a block diagram of a stereoscopic real time or static image viewing system where the operational sequence is shown for a first alternative embodiment of the invention herein.
Figure 7 shows a block diagram of a stereoscopic real time or static image viewing system where the operational sequence for a second alternative embodiment of the invention herein is shown.
Figure 8A shows a first configuration of the second image modifying unit used in the embodiment illustrated in Figure 7.
Figure 8B shows a second configuration of the second image modifying unit used in the embodiment illustrated in Figure 7.
Figure 9 shows a block diagram of a stereoscopic real time or static image viewing system where the operational sequence for a third alternative embodiment of the invention herein is shown.

### DETAILED DESCRIPTION OF THE INVENTION

It has been found that by the combination of various devices, it is possible to obtain a stereoscopic real time or static image viewing system that shows a real image obtained from capturing means of images at the moment at which they are generated, such as video cameras, endoscopes in their different varieties, surgical microscope, photographic cameras, or any other system for obtaining video or printed images in real time or from images previously obtained and stored, with a simple yet very efficient three-dimensional viewing effect that permits the observer to perform fine and precise movements with adequate perception of volume, distance and depth.

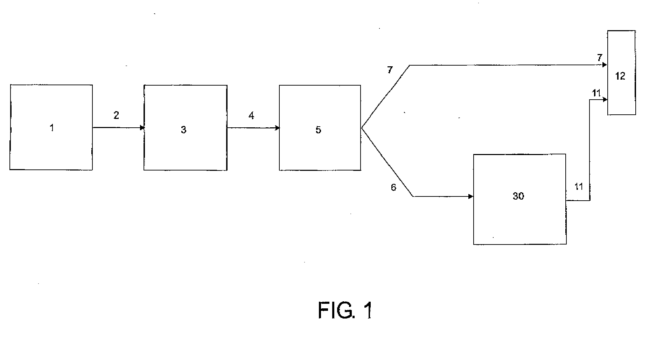

With particular reference to the attached drawings and more specifically to their Figure 1, it shows a block diagram that indicates the operational sequence of the stereoscopic real time or static image viewing system built in accordance with a particularly specific embodiment of the invention herein and representing them from obtention of the images until they reach the eyes of the observer. The system comprises in general terms in association: first image capturing means 1 for capturing at least one original image 2, either with or without movement; conversion means 3 for (converting) images to digital and/or analog signals, which receive the original image 2 and convert it to an original image signal 4; image signal duplication means 5, which receive the original image signal 4 to generate simultaneously two image signals, a first duplicated image signal 6 and a second duplicated image signal 7; a first image modifying unit 30, which, from the first duplicated image signal 6 generates a first modified image signal 11 that consists of the original image 2 under a perspective that differs from the one by means of which it was originally captured by the first image capturing means 1; and three-dimensional viewing means 12, that receive the signals from the second duplicated image 7 and from the first modified image 11 to achieve the three-dimensional viewing of the original image 2 by means of the combination of an image obtained from the second duplicated image signal 7 and from a modified image obtained from the first modified image signal 11.

The first image capturing means 1 are selected from among activities or events that occur live, using for this purpose video cameras, surgical microscopes, photographic cameras, ultrasound, navigators, endoscopes in their different varieties, preferably neuroendoscopes, endoscopes, thoraxoscopes laparoscopes, pelviscopes, arthroscopes, three-dimensional endoscopes (E-3D), or any other system for obtaining video and/or printed images.

As far as the means 3 for converting images to digital and/or analog signals are concerned, as well as the image signal duplication means 5, it should be noted that these means are selected from among the various elements and devices that are widely known in the field of electronics, so that, for example, the image signal duplication means 5 may be a "Y" type signal divider. Likewise, in the specific embodiment that is described in the invention herein, it is contemplated that these conversion means 3 and the image duplication means 5 can be included in the first image capturing means 1.

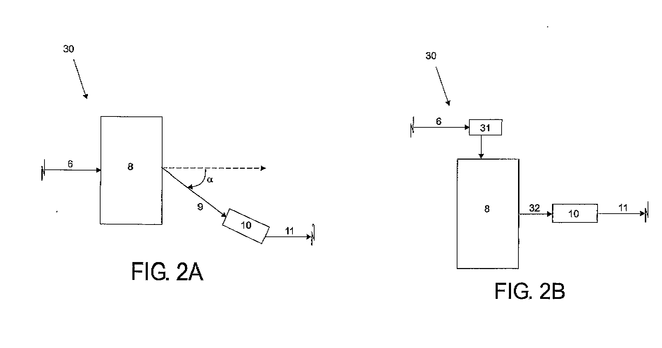

In order to clearly explain an important part of the stereoscopic viewing system of the invention herein, reference is now made to Figure 2A, which shows a first configuration of the first image modifying unit 30 that comprises: First image projection means 8 that receive the first duplicated image signal 6 for projecting a first duplicated image 9; and second image capturing means 10 for capturing this duplicated image 9 from a first oblique visual angle of incidence □ with reference to the normal of the projection surface of these first image projection means 8; these second image capturing means 10 generate the first modified image signal 11.

As can be observed from the above, the capturing of the first duplicated image 9 from this first oblique visual angle of incidence □ permits obtaining a perspective of the original image 2 that differs from the one captured by the first image capturing means 1.

As far as the first image projection means 8 are concerned, they are selected from among video screens, with or without kinescope, liquid crystal screens (LCD), plasma screens or video projection screens, preferably with a flat surface, on which an image is projected by means of a video projector. Likewise, the second image capturing means 10 are selected from among video or digital cameras.

For better capturing the first duplicated image 9, it is preferred that it as well as the first image projection means 8 and the second image capturing means 10 are located within a relatively hermetic and light-insulated environment.

Related to the first oblique visual angle of incidence □, it has a value between 0° and 90°, and more specifically, it has a value from 6 to 30°.

In an alternative embodiment of the invention herein, the first image modifying unit 30 presents a second configuration, as can be appreciated in Figure 2B of the attached drawings that comprises: The first image projection means 8, in which are integrated the first image editing means 31, which generate, from the first original image signal 6, a first edited image 32 that is projected on these first image projection means 8. The first edited image 32, which consists of the original image 2 with an effect provided by the first image editing means 31 that appears to have been captured from a perspective that differs from that with which it was originally captured; and the second image capturing means 10 placed in front of the first image projection means 8 to capture the first edited image 32 and to generate this first modified image signal 11.

To achieve better comprehension of the first edited image 32 achieved as a result of the first image editing means 31, it is important to mention that this first edited image 32 may be perceived in the first image projection means 8 in such a way that its height becomes reduced from the left to the right, or vice versa, of the projection surface, and in the direction toward a vanishing point. On the other hand, it has also been contemplated that the first image editing means 31 directly generate the first modified image signal 11.

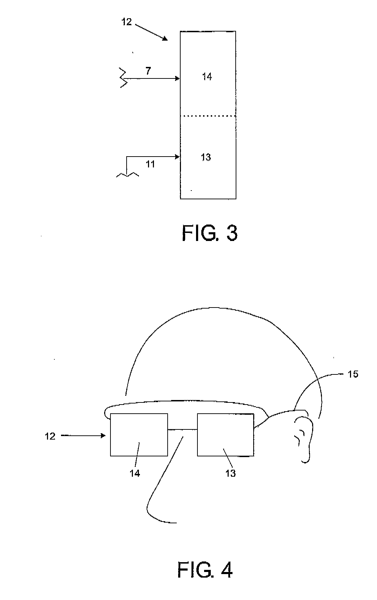

The three-dimensional viewing means 12 that are described and shown in Figure 3 of the attached drawings comprise a first and a second image projection element 13 and 14 respectively, where the first image projection element 13 permits the viewing of the original image that has been modified in its perspective and is obtained from the first modified image signal 11, while the second projection element 14 permits viewing the original image 2 obtained from the second duplicated image signal 7. It is noteworthy that the first 13 and second 14 image projection elements are selected from among liquid crystal screens, plasma screens or screens with kinescope, or any other image projection means.

In the particularly preferred embodiment that is described, the first 13 and second 14 image projection elements can be mounted on a cephalic and/or facial placement support 15 similar to the frame of certain eyeglasses, which additionally permits the user to move the head freely without losing the three-dimensional feeling, permits maintaining the "eye- hand" relation and observing the images for extended periods of time and directly in front of the eyes, as shown in Figure 4 of the attached drawings.

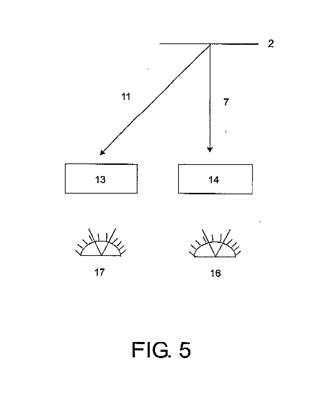

Then, to understand the operation of the three-dimensional viewing means 12 of the stereoscopic viewing system of the invention herein, Figure 5 of the attached drawings shows a schematic diagram that simulates the visual angles of incidence perceived by an observer placed in front of the three-dimensional viewing means 12 of Figure 4, where it is evident that, using an eye 16, the observer can see the original image 2 projected on the second image projection element 14, while, with the opposite eye 17, perceiving in the first image projection element 13 the same original image 2 under a perspective that differs from the one with which it was captured.

Likewise, in another alternative embodiment of the invention herein, the image signals 7 and 11 are multiplied as many times as desired, in order to provide several observers the ability to see them simultaneously on other independent three-dimensional viewing means.

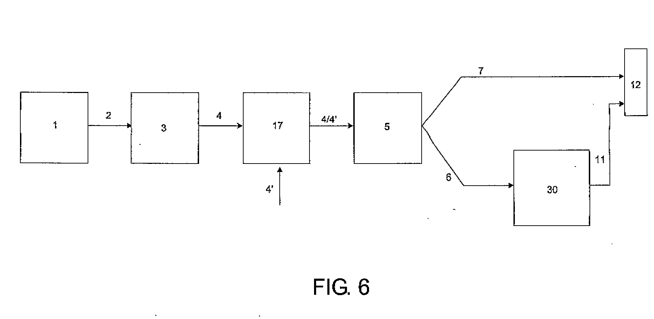

On the other hand, with specific reference to Figure 6 of the attached drawings, it shows a block diagram that indicates the operational sequence of the stereoscopic real time or static image viewing system in accordance with a first alternative embodiment of the invention herein, representing them from obtention of the images through their arrival at the eyes of the observer. As can be observed from Figure 6, this embodiment is very similar to the embodiment described in Figure 1; however, the stereoscopic viewing system additionally comprises: image mixing and selecting means 17 that receive the original image signal 4 and mix it with an auxiliary image signal 4' that contains at least one auxiliary image (not shown in the figures), these image mixing and selecting means 17 generating a signal of images in boxes 4/4' ("picture in picture") integrated by the original image signal 4 and the auxiliary image signal 4'; this signal of images in boxes 4/4' is processed later by the rest of the system in the same way in which the original image signal 4 is processed in the embodiment shown in Figure 1, thus achieving the three-dimensional viewing in boxes of the original image 2 and of the auxiliary image.

In order to explain the above in detail, in this embodiment, this signal of images in boxes 4/4' is received by these image duplicating means 5 that simultaneously generate two image signals, a first duplicated image signal 6 and a second duplicated image signal 7, the signal of the first duplicated image 6 being received by the first image modifying unit 30, which generates a first modified image signal 11 which, for this embodiment, consists of a combination in boxes of the original image 2 and of the auxiliary image, both under a perspective that differs from the one with which they were originally captured; the first modified image signal 11 and the second duplicated image signal 7 are received by these three-dimensional viewing means 12, in which three-dimensional viewing is achieved and in boxes the original image 2 and the auxiliary image by means of the combination of an image in boxes obtained from the second duplicated image signal 7 and from an image in boxes modified in their perspective obtained from the first modified image signal 11.

The image selecting and mixing means 17 can be a conventional type video mixer. Likewise, it is noteworthy that the auxiliary image signal 4' can be obtained or generated by image capturing means (not shown in the figures) similar to those described above for the specific embodiment of the invention herein shown in Figure 1.

To explain the operation of the stereoscopic viewing system described in the first alternative embodiment of the invention herein and the way in which the user captures the images processed by the system, it should be mentioned that during the surgical procedure, a video camera captures the image of the surgical area and generates an original image signal 4, while the auxiliary signal 4' can be generated by an endoscope in the same surgical area, or rather, is a signal that contains a series of stored images from a transoperative study, such as a doppler, a navigation system, etc. Later, when this image signal in boxes 4/4' is generated and is to be processed by the system of the invention herein, until arrival at the three-dimensional viewing means 12, a user can three-dimensionally perceive both images like images in boxes (one main image taken by the video camera and a secondary image captured by the endoscope, or vice versa).

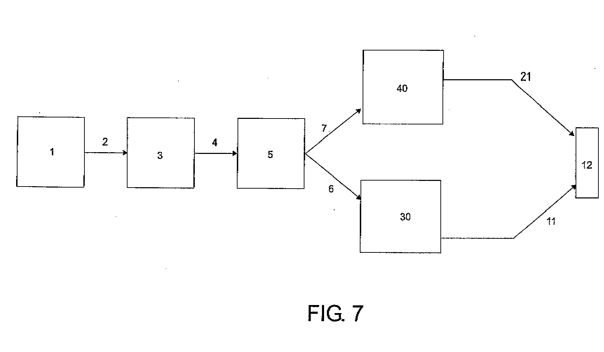

On the other hand, Figure 7 of the attached drawings shows a block diagram that indicates the operational sequence of the stereoscopic real time or static image viewing system in accordance with a second alternative embodiment of the invention herein, representing it from obtention of the images to their arrival at the eyes of the observer. In the second alternative embodiment that is described, it is possible to observe that the processing of the second duplicated image signal 7 is different from the one of the specific embodiment shown in Figure 1, since in this second alternative embodiment the stereoscopic viewing system additionally comprises: a second image modifying unit 40, which, from the second duplicated image signal 7 generates a second modified image signal 21 that consists of the original image 2 under a perspective that differs both from the one that with which it was captured by the first image capturing means 1 as well as being different from the perspective achieved by means of the first image modifying unit 30 with which, in the three-dimensional viewing means 12, the first and the second modified image signals 11 and 21 respectively are received, to achieve the three-dimensional viewing of the original image 2 by means of the combination of a first modified image obtained from the first duplicated image signal 11 and a second modified image obtained from the second modified image signal 21.

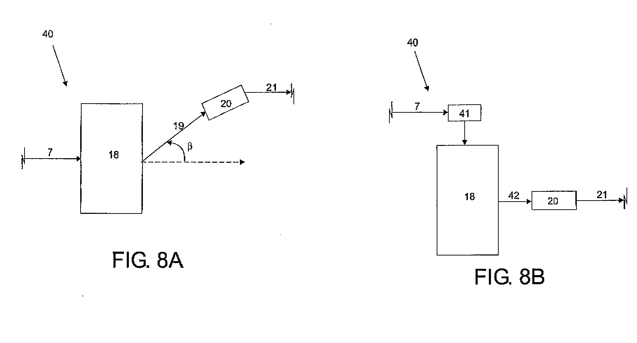

The second image modifying unit 40 in a first configuration shown in Figure 8A of the attached drawings comprises: second image projection means 18 that receive the second duplicated image signal 7 to project a second duplicated image 19; and third image capturing means 20 for capturing this second duplicated image 19 under a second oblique visual angle of incidence □ with reference to the normal of the projection surface of these second image projection means 18, these third image capturing means 20 generating this second modified image signal 21. In this respect, the second oblique visual angle of incidence □ differs from the first visual angle of incidence □ in the first image modifying unit 30 when the same has the configuration shown in Figure 2A.

In the second alternative embodiment that is described in the invention herein, the second image projection means 18 and the third image capturing means 20 are similar to the first projection means 8 and the second capturing means 10 described above. These second projection means 18 and third capturing means 20 together with the second duplicated image 19 are preferably located in a relatively hermetic and light-insulated environment. Related to the angle □, its value is within the limits indicated and preferred for the angle □ but subject to the condition that the value of each angle is different from the other.

Likewise, the second image modifying unit 40 in a second configuration shown in Figure 8B of the attached drawings comprises: Second image projection means 18 integrated into second image editing means 41 which generate from the second original image signal 7 a second edited image 42 that is projected on these second image projection means 18. The second edited image 42 consists of the original image 2 with an effect provided by the second image editing means 41 in such a way that the original image 2 appears to have been captured under a different perspective with respect to the one under which it was originally captured and is also different from the one achieved by the first image modifying unit 30 in any of its two configurations; and third image capturing means 20 placed in front of the second image projection means 18 to capture the second edited image 42 and generate the second modified image signal 21. It should be stressed that the second image editing means 31 can also generate this second modified image signal 21.

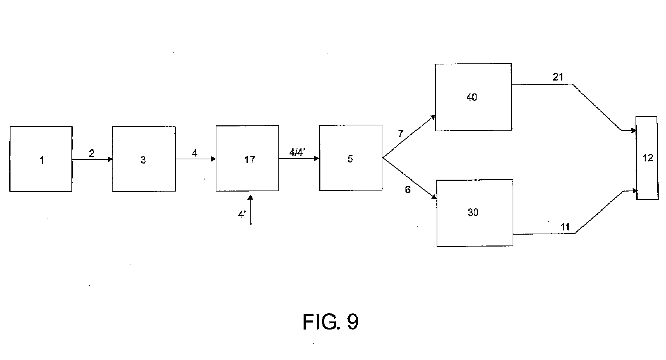

Finally, Figure 9 of the attached drawings shows a block diagram that indicates the operational sequence of a stereoscopic real time or static image viewing system in accordance with a third alternative embodiment of the invention herein, which, in addition to including all the elements described in the second alternative embodiment and illustrated in Figure 7, comprises: image mixing and selecting means 17 which receive the original image signal 4 and mix it with an auxiliary image signal 4' which contains at least one auxiliary image, these image mixing and selecting means 11 generating a signal of images in boxes 4/4' (picture in picture) integrated by the original image signal 4 and the auxiliary image signal 4', this signal of images in boxes 44 being duplicated to obtain a first duplicated image signal 6 and a second duplicated image signal 7, which are processed accordingly as described in the embodiments shown in Figures 1 and 7 respectively and thus achieve the three-dimensional viewing in boxes of the original image 2 and of the auxiliary image.

In order to explain the above in detail, in this embodiment, the first duplicated image signal 6 is received by the first image modifying unit 30, which generates a first modified image signal 11 that consists of a combination in boxes of the original image 2 and of the auxiliary image, both under a perspective that differs from the one under which they were originally captured; the second duplicated image signal 7 is received by the second image modifying unit 40 that generates a second modified image signal 21 which consists of a combination in boxes of an original image 2 and of the auxiliary image, both under a perspective that differs from both the one under which they were originally captured and different from the perspective that is achieved by means of the first image modifying unit 30, by means of which, in the three-dimensional viewing means 21, the first 11 and second 21 modified image signals are received to achieve the three-dimensional viewing of the original image 2 and of the auxiliary image by means of the combination in boxes of two modified images obtained from the image signals 11 and 21, each signal offering a perspective that differs from the original image 2 and the auxiliary image.

In the third alternative embodiment that is described, it will be evident that these image mixing means 4 are identical to those described above for the first alternative embodiment shown in Figure 6.

In accordance with the description above, it will be possible to observe that the stereoscopic real time or static image viewing system has been conceived for simple but at the same time efficient three-dimensional image viewing that can be used to observe any type of images for purposes other than medical and industrial, and it will be obvious to any man of the art that the embodiments of the stereoscopic real time or static image viewing system described above and illustrated in the attached drawings, are only for illustration purposes and not limitative in the invention herein, because numerous changes of consideration in their details are possible but without deviating from the scope of the invention.

Although certain embodiments of the invention have been illustrated and described, it must be stressed that numerous modifications to it are possible, such as the projection means of the second duplicated image 7, different angles □ and □ for capturing the duplicated images with respect to the projection surface of the image projection means, combining configurations of the first and second image modifying unit 30 and 40 respectively, or different adaptations of the three-dimensional viewing means 12, among others. Therefore, the invention herein should not be considered as being restricted except by whatever is required by the technique above and by the scope of the attached claims.

## Claims

1. A stereoscopic real time or static image viewing system **characterized in that** it comprises: first image capturing means (1) for capturing at least one original image (2), either with or without movement; conversion means (3) for (converting) images to digital and/or analog signals, which receive the original image (2) and convert it to an original image signal (4); image signal duplicating means (5) which receive the original image signal (4) to simultaneously generate two image signals, a first duplicated image signal (6) and a second duplicated image signal (7); a first image modifying unit (30), which generates from the first duplicated image signal (6) a first modified image signal (11) that consists of the original image (2) under a perspective that differs from the one with which it was originally captured by the first image capturing means (1); and three-dimensional viewing means (12) that receive the second duplicated image signal (7) and the first modified image signal (11) to achieve the three-dimensional viewing of the original image (2) by combining an image obtained from the second duplicated image signal (7) and a modified image obtained from the first modified image signal (11).

2. A stereoscopic real time or static image viewing system in accordance with Claim 1, moreover **characterized in that** it comprises the first image capturing means (1) that are selected from among activities or events that occur live, using for this purpose video cameras, surgical microscopes, photographic cameras, ultrasound, navigators, endoscopes, or any other system for obtaining video and/or printed images.

3. A stereoscopic real time or static image viewing system in accordance with Claim 2, moreover **characterized in that** the endoscopes are selected from among neuroendoscopes, endoscopes, thoraxoscopes, laparoscopes, pelviscopes, arthroscopes, three-dimensional endoscopes (E-3D).

4. A stereoscopic real time or static image viewing system in accordance with Claim 1, moreover **characterized in that** the image signal duplicating means (5) are a "Y" type signal divider.

5. A stereoscopic real time or static image viewing system in accordance with Claim 1, moreover **characterized in that** the digital and/or analog image signal conversion means (3) and the image signal duplication means (5) are included in the first image capturing means (1).

6. A stereoscopic real time or static image viewing system in accordance with Claim 1, moreover **characterized in that** the first image modifying unit (30) comprises: first image projection means (8), which receive the first duplicated image signal (6) to project a first duplicated image (9); and second image capturing means (10) for capturing this duplicated image (9) under a first oblique visual angle of incidence □ with respect to the normal of the projection surface of these first image projection means (8), these second image capturing means (10) generating the first modified image signal (11).

7. A stereoscopic real time or static image viewing system in accordance with Claim 6, moreover **characterized in that** the first image projection means (8) are selected from among video screens with or without kinescope, liquid crystal screens (LCD), plasma screens, or video projection screens, onto which an image is projected by means of a video projector.

8. A stereoscopic real time or static image viewing system in accordance with Claim 7, moreover **characterized in that** the video projection screens have a flat surface.

9. A stereoscopic real time or static image viewing system in accordance with Claim 6, moreover **characterized in that** the second image capturing means (10) are selected from among video or digital cameras.

10. A stereoscopic real time or static image viewing system in accordance with Claim 6, moreover **characterized in that** the first duplicated image (9) as well as the first image projection means (8) and the second image capturing means (10) are located in a relatively hermetic and light-insulated environment.

11. A stereoscopic real time or static image viewing system in accordance with Claim 6, moreover **characterized in that** the first oblique visual angle of incidence □ has a value between 0° and 90°.

12. A stereoscopic real time or static image viewing system in accordance with Claim 11, moreover **characterized in that** the first oblique visual angle of incidence □ has a value from 6° to 30°.

13. A stereoscopic real time or static image viewing system in accordance with Claim 1, moreover **characterized in that** the first image modifying unit (30) comprises: first image projection means (8) integrated into first image editing means (31) which generate from the first original image signal (6) a first edited image (32) that is projected onto these first image projection means (8); and second image capturing means (10) placed in front of the first image projection means (8) to capture the first edited image (32) and generate the first modified image signal (11), the first edited image (32) consisting of the original image (2) with an effect provided by the first image editing means (31), so that it appears to have been captured under a different perspective with respect to the one by means of which it was originally captured.

14. A stereoscopic real time or static image viewing system in accordance with Claim 13, moreover **characterized in that** the first editing means (31) directly generate the first modified image signal (11).

15. A stereoscopic real time or static image viewing system in accordance with Claim 1, moreover **characterized in that** the three-dimensional viewing means (12) comprise: a first image projection element (13) and a second image projection element (14) where the first image projection element (13) permits the viewing of the original image that has been modified in its perspective and which is obtained from the first modified image signal (11), while the second of projection element (14) permits viewing of the original image (2) obtained from the second duplicated image signal (7).

16. A stereoscopic real time or static image viewing system in accordance with Claim 15, moreover **characterized in that** the first (13) and the second (14) image projection elements are selected from among liquid crystal screens, plasma screens or screens with kinescope, or any other image projection means.

17. A stereoscopic real time or static image viewing system in accordance with Claim 15, moreover **characterized in that** the first (13) and the second (14) image projection elements are mounted on a cephalic and/or facial placement support (15) similar to the frame of certain eyeglasses, which, in addition to permitting the users to move their head freely without losing the three-dimensional feeling, permits maintaining the "eye-hand" relation and observing the images for extended periods of time and directly in front of the eyes.

18. A stereoscopic real time or static image viewing system in accordance with Claim 17, moreover **characterized in that** an observer who is placed in front of the three-dimensional viewing means (12) sees with one eye (16) the original image (2) projected on the second image projection element (14), while with the opposite eye (17) perceiving in the first image projection element (13) the same original image (2) under a perspective that differs from the one under which it was originally captured.

19. A stereoscopic real time or static image viewing system in accordance with Claim 1, moreover **characterized in that** the second duplicated image signal (7) and the first modified image signal (11) are multiplied as many times as desired to provide several observers the ability to see them simultaneously in other independent three-dimensional viewing means.

20. A stereoscopic real time or static image viewing system in accordance with Claim 1, moreover **characterized in that** this system additionally comprises: image mixing and selecting means (17) that receive the original image signal (4) and mix it with an auxiliary image signal (4') that contains at least one auxiliary image, these image mixing and selecting means (17) generating an image signal in boxes (4/4') ("picture in picture") that is integrated by this original image signal (4) and this auxiliary image signal (4'), this signal of images in boxes (4/4') being later received by these image duplicating means (5) that simultaneously generate two image signals, a first duplicated image signal (6) and a second duplicated image signal (7), the first duplicated image signal (6) being received by the first image modifying unit (30), which generates a first modified image signal (11) that consists of a combination in boxes of the original image (2) and of the auxiliary image, both under a perspective that differs from the one under which they were originally captured, the first modified image signal (11) and the second duplicated image signal (7) being received by these three-dimensional viewing means (12) in which three-dimensional viewing is achieved and in boxes the original image (2) and the auxiliary image by means of the combination in boxes of an image obtained from the second duplicated image signal (7) and from an image in boxes modified in its perspective obtained from the first modified image signal (11).

21. A stereoscopic real time or static image viewing system in accordance with Claim 20, moreover **characterized in that** the image selecting and mixing means (17) are a conventional type video mixer.

22. A stereoscopic real time or static image viewing system in accordance with Claim 20, moreover **characterized in that** the auxiliary image signal (4') is obtained or generated by image capturing means that are selected from among activities or events that occur live, video cameras, surgical microscopes, photographic cameras, ultrasound, navigators, endoscopes, or any other system for obtaining video and/or printed images.

23. A stereoscopic real time or static image viewing system in accordance with Claim 1, moreover **characterized in that** the stereoscopic real time or static image viewing system additionally comprises: a second image modifying unit (40), which generates, from the second duplicated image signal (7) a second modified image signal (21) that consists of the original image (2) under a perspective that differs from both the one by means of which it was captured by the first image capturing means (1) and is different from the perspective that is achieved by means of the first image modifying unit (30) by means of which, on the three-dimensional viewing means (12), the first (11) and second (21) modified image signals are received to achieve the three-dimensional viewing of the original image (2) by means of the combination of a first modified image obtained from the first duplicated image signal (11) and a second modified image obtained from the second modified image signal (21).

24. A stereoscopic real time or static image viewing system in accordance with Claims 6 and 23, moreover **characterized in that** the second image modifying unit (40) comprises: second image projection means (18) that receive the second duplicated image signal (7) to project a second duplicated image (19); and third image capturing means (20) for capturing this second duplicated image (19) under a second oblique visual angle of incidence □ with respect to the normal of the projection surface of these second image projection means (18) where these third image capturing means (20) generate this second modified image signal (21), and this second oblique visual angle of incidence □ differs from the first visual angle of incidence □ in the first image modifying unit (30).

25. A stereoscopic real time or static image viewing system in accordance with Claim 24, moreover **characterized in that** the second image projection means (18) are selected from among video screens with or without a kinescope, liquid crystal screens (LCD), plasma screens, or video projection screens, on which an image is projected by means of a video projector.

26. A stereoscopic real time or static image viewing system in accordance with Claim 25, moreover **characterized in that** the video projection screens have a flat surface.

27. A stereoscopic real time or static image viewing system in accordance with Claim 24, moreover **characterized in that** the third image capturing means (20) are selected from among video or digital cameras.

28. A stereoscopic real time or static image viewing system in accordance with Claim 24, moreover **characterized in that** the second projection means (18) and the third image capturing means (20) together with the second duplicated image (19) are located in a relatively hermetic and light-insulated environment.

29. A stereoscopic real time or static image viewing system in accordance with Claim 24, moreover **characterized in that** the second oblique visual angle of incidence □ has a value between 0° and 90°.

30. A stereoscopic real time or static image viewing system in accordance with Claim 29, moreover **characterized in that** the second oblique visual angle of incidence □ has a value from 6° to 30°.

31. A stereoscopic real time or static image viewing system in accordance with Claim 24, moreover **characterized in that** the second image modifying unit (40) comprises: second image projection means (18) integrated into second image editing means (41), which generate from the second original image signal (7) a second edited image (42) that is projected on these second image projection means (18) where the second edited image (42) consists of the original image (2) with an effect provided by the second image editing means (41) in such a way that the original image (2) appears to have been captured under a different perspective with respect to the one under which it was originally captured; and the third image capturing means (20) placed in front of the second image projection means (18) to capture the second edited image (42) and generate the second modified image signal (21).

32. A stereoscopic real time or static image viewing system in accordance with Claim 23, moreover **characterized in that** the stereoscopic real time or static image viewing system additionally comprises: image mixing and selecting means (17) that receive the original image signal (4) and mix it with an auxiliary image signal (4') that contains at least one auxiliary image, where these image mixing and selecting means (17) generate a signal of images in boxes (4/4') (picture in picture) integrated by the original image signal (4) and the auxiliary image signal (4'), this signal of images in boxes (4/4') being later received by these image duplication means (5) that simultaneously generate two image signals, a first duplicated image signal (6) and a second duplicated image signal (7), the first duplicated image signal (6) being received by the first image modifying unit (30), which generates from the first duplicated image signal (6) a first modified image signal (11) that consists of a combination in boxes of the original image (2) and the auxiliary image, both under a perspective that differs from the one under which they were originally captured, the second duplicated signal being received by the second image modifying unit (40) that generates a second modified image signal (21) that consists of a combination in boxes of the original image (2) and the auxiliary image, both under a perspective that differs both from the one by means of which they were originally captured and different from the perspective that is achieved by means of the first image modifying unit (30), by means of which, in the three-dimensional viewing means (12), the first (11) and second (21) modified image signals are received to achieve three-dimensional viewing of the original image (2) and of the auxiliary image by means of the combination of two images in modified boxes obtained from the image signals (11) and (21), each signal offering a perspective that differs from the original image (2) and the auxiliary image.
